# EUROPEAN PATENT APPLICATION

(11) **EP 3 459 496 A1**
(43) Date of publication of application: **27.03.2019**
(21) Application number: 18275138.8
(22) Date of filing: 03.09.2018
(51) Int. Cl.: A61F 2/07, A61F 2/06

(54) **ENDOVASCULAR STENT GRAFT WITH COAGULATION PROMOTING FEATURE**

(30) Priority: 20.09.2017 US 201715709638
(71) Applicant: Cook Medical Technologies LLC, Bloomington, IN 47404 (US)
(72) Inventor: Baxter, Charles L., West Lafayette, IN Indiana 47906 (US); KRATZBERG, Jarin, Lafayette, IN Indiana 47909 (US)
(74) Representative: Williams Powell

(57) **Abstract**

A stent graft (20) includes a fabric tube (22) supported by and attached to a stent (21). A plurality of streamers (30) are attached at one end to an outer surface of the fabric tube (22) and have an unattached coagulant end that is free to move with respect to the fabric tube (22) within a sac of an aneurism to promote coagulation of blood, such as to inhibit Type II endoleaks. Each of the streamers (30) has a length dimension that is greater than both a width dimension and thickness dimension. The streamers (30) may comprise at least one of a extracellular matrix material, a hydrogel and embolic fibers.

## Description

### Technical Field

The present disclosure relates generally to stent grafts, and more particularly to stent grafts for treating aneurisms that may be at risk for Type II endoleaks.

### Background

A Type II endoleak is a known challenge in stent graft based aneurism treatment and has prompted a number of complex approaches using combinations of existing technology and proactive treatment schemes, all of which are undesirable but often necessary. In some instances re-intervention for the treatment of Type II endoleaks can be extremely challenging and risky due to stent graft placement and the obstruction of access to the aneurism sac feeding vessels.

The present disclosure is directed toward one or more of the problems set forth above.

### Summary

The present invention seeks to provide an improved stent graft. There is also described an improved method of treating an aneurism.

According to an aspect of the present invention, there is provided a stent graft comprising a fabric tube supported by and attached to a stent. A plurality of streamers each include an attachment end attached to an outer surface of the fabric tube and an unattached coagulant end that is free to move with respect to the fabric tube. The streamers promote coagulation of blood that contacts the streamers after the stent graft is implanted. Each of the streamers has a length dimension that is greater than both a width dimension and a thickness dimension.

A method of treating an aneurism includes the steps of implanting a stent graft at the aneurism, wherein coagulation of blood in the sac of the aneurism outside of the stent graft is encouraged with streamers attached at one end to the stent graft. The streamers comprise at least one of an extracellular matrix material, a hydrogel and embolic fibers.

### Brief Description of the Drawings

Embodiments of the present invention are described below, by way of example only, with reference to the accompanying drawings, in which:
Fig. 1 is a side schematic view of a stent graft according to the present disclosure implanted at an aneurism implantation site; and
Fig. 2 is an enlarged schematic perspective view of a streamer from the stent graft of Fig. 1.

### Detailed Description

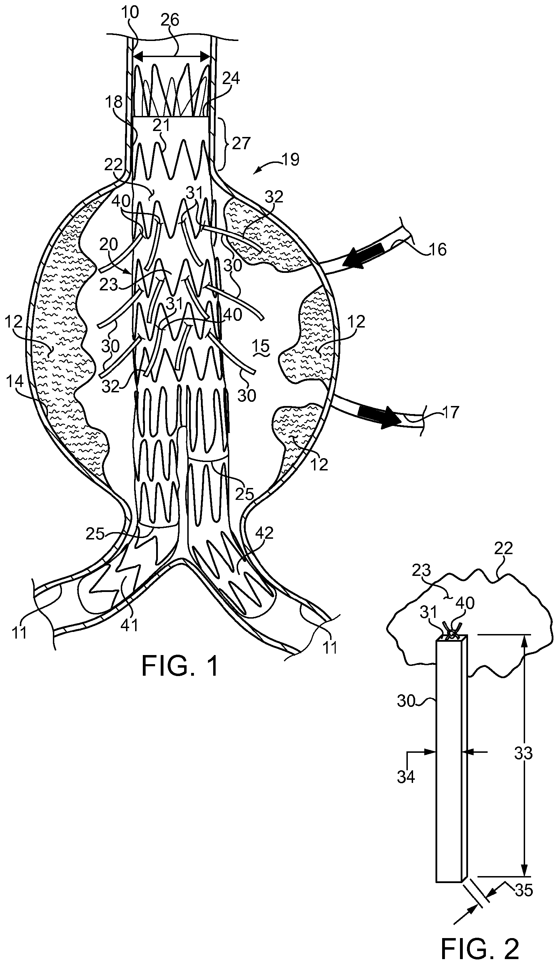

Referring to Figs. 1 and 2, a stent graft 20 is shown with a pair of secondary or leg stent grafts 41 and 42 spanning an aneurism 14 in an aorta 10 just above the pair of illiac arteries 11. Stent graft 20 includes a fabric tube 22 supported by and attached to a stent structure 21. As used in this disclosure, the term "fabric" means any of a wide variety of medical grade permeable and non-permeable flexible sheets that may be made up of plastic films and/or woven or unwoven fibers or other structures known in the art. Stent 21 is illustrated as a self expanding stent 21, but a balloon expanded stent could also fall within the scope of the present disclosure. A plurality of streamers 30 each includes an attachment end 31 attached to an outer surface 23 of the fabric tube 22, and an unattached coagulant end 32 that is free to move with respect to the fabric tube 22. The attachment ends are out of contact with each other and therefore the streamers 30, when considered as a group, cannot properly be considered as a "fluff layer" as described in WO-2016/095864. In other words, a "plurality of streamers" according to the present disclosure means something other than the PLGA fluff layer taught in WO-2016/095864. furthermore, individual streamers means something other than an individual piece of the fluff layer taught in WO-2016/095864. The streamers promote coagulation of blood that contacts the streamers after the stent graft 20 is implanted. Each of the streamers has a length dimension 33 that is greater than both a width dimension 34 and a thickness dimension 35 thereof. The streamers 30 may be attached directly to the fabric tube using individual sutures 40, or may be attached with sutures to the stent 21, and hence indirectly attached to the fabric tube 22. In addition, those skilled in the art will appreciate that any suitable attachment strategy would fall within the scope of the present disclosure.

Although the illustrated stent graft 20 has exactly one entrance opening 24 and two exit openings 25, those skilled in the art will appreciate that stent grafts having any number of entrance openings and exit openings, including a single entrance and single exit, would fall within the intended scope of the present disclosure. Preferably, the length dimension 33 of the streamers 30 is of a same order of magnitude as an entrance diameter 36 at the entrance opening 24 of the fabric tube 22. Those skilled in the art will appreciate that the term "same order of magnitude" means that neither dimension is more that ten times the other dimension. The range of lengths 33 of the streamers 30 could vary, but should be considered carefully to help promote durability of tethering of the streamers in order to avoid potential downstream embolic events. As the streamers 30 are intended to promote coagulation of blood in the sac 15 of aneurism 14, they preferably have width and thickness dimensions that allow for some substantial flexibility so that the coagulant end can drift away from fabric tube 22 and float in contact with blood within the sac 15. For instance, streamers might promote coagulation of blood that may be flowing through or into sac 15 due to blood vessels 16 and 17, which might otherwise result in a Type II endoleak. In the illustrated embodiment, some established clot formation 12 already exists in sac 15, but some flow may continue due to blood vessels 16 and 17. With the help of streamers 30, the complete sac 15 may be filled with coagulated blood in order to stop blood vessel 16 and 17 from feeding sac 15.

The streamers 30 are preferably manufactured from a suitable coagulant promoting material. These materials include, but are not limited to one or a combination of: extra cellular matrix materials, hydrogels and embolic fibers. In the case of an extra cellular matrix, the streamer may be constructed from small intestine submucosa. If the streamers 30 include embolic fibers, the embolic fibers may be constructed as a woven ribbon that includes the embolic fibers. The chosen material can also be cast or compressed hydrogel strips. Although not necessary, the streamers 30 may include a rapidly dissolvable coating to inhibit coagulation of blood prior to the treatment procedure being complete. In preferred embodiments, a dissolvable coating may have a thickness designed to dissolve within two to three times the expected treatment duration to ensure that the streamers do not promote coagulation of blood anywhere in the patient other than outside of the fabric tube 22 but within the sac 15 of aneurism 14.

Although not necessary, an end segment of the stent graft 20 may be considered a sealing segment 27 for contacting vessel wall 18 at the implantation site 19. In the illustrated embodiment, the sealing segment 27 is free of streamer attachments, and all of the streamers 30 are attached to the fabric tube 22 away from sealing segment 27. This strategy may help prevent less than adequate sealing between sealing segment 27 and the vessel wall 18.

The number of streamers and their specific dimensions and distribution on the fabric tube are a matter of design choice. Preferably, the streamers are attached 360° around the fabric tube 22 and distributed along the length of fabric tube to quickly promote coagulation of blood in sac 14 to otherwise inhibit complications, such as Type 2 endoleaks that might otherwise occur.

### Industrial Applicability

The present disclosure finds potential application in any stent graft. The present disclosure finds more specific application to stent grafts used for treating aneurisms. Finally, the present disclosure finds particular application to stent grafts used for aortic aneurism treatments. Although the present disclosure is illustrated in the context of treatment of an aortic aneurism, those skilled in the art will appreciate that the stent graft 20 of the present disclosure may also be implanted in an artificial passageway, such as for teaching or demonstration purposes without departing from the intended scope of the present disclosure.

In one specific example, a method of treating an aneurism 14 includes implanting a stent graft 20 at the aneurism 14. Coagulation of blood in the sac 15 of the aneurism 14 that is outside of the stent graft 20 is encouraged with streamers 30 that are attached at one end to the stent graft 20. Each of the streamers 30 preferably comprises at least one of an extracellular matrix material, a hydrogel and embolic fibers. The present disclosure teaches preferably inhibiting contact between blood and the coagulant promoting material of streamers 30 during the implanting step by covering the streamers 30 with a dissolvable coating. In that way, during the stenting procedure, blood in the patient's circulatory system is not exposed to coagulant promoting materials. The dissolvable coating may be designed to have a thickness such that one could expect the coating to dissolve in a duration of time equal to about two or three times the expected implantation procedure duration. In this way, one could expect the coagulant promoting properties of stent graft 20 to not begin until potentially hours after the aneurism treatment procedure is completed. Preferably, the streamers 30 are isolated from contact with the vessel wall 18 by locating the streamers 30 away from the sealing segment 27 of stent graft 20.

By choosing materials and geometry for the streamers 30 in the appropriate manner, they ban be allowed to float in the aneurism sac 15 and be agitated by blood flow in the sac following device deployment. This can be advantageous because contact between the thrombolytic or coagulant promoting material and uncoagulated blood in the sac will help promote coagulation within the sac 15. If coagulation can be promoted in an open area around the stent graft 20, then the supply vessels 16 and 17 that may contribute to continued expansion of the aneurism 14 can be reduced or stopped by leveraging the action of the clotting cascade produced by the streamers 30.

One advantage of this concept is to provide a potential solution for Type II endoleaks without the burden of secondary intervention or the placement of additional devices preemptively to avoid a Type II failure mode. The impact of avoiding secondary inventions is extremely significant because of the risks associated with any secondary intervention.

As will be appreciated, there is also disclosed herein a method of treating an aneurism, comprising the steps of: implanting a stent graft at the aneurism; encouraging coagulation of blood in the sac of the aneurism outside of the stent graft with streamers attached at one end to the stent graft; and wherein the streamers comprise at least one of a extracellular matrix material, a hydrogel and embolic fibers.

The method advantageously includes temporarily inhibiting contact between blood and the at least one of the bioscaffold, the hydrogel and the embolic fibers during the implanting step by covering the streamers with a dissolvable coating.

The encouraging step preferably includes sizing a length dimension of the streamers to be of a same order of magnitude as an entrance diameter of the stent graft.

The method preferably includes isolating the streamers from contact with a vessel wall by locating the streamers away from a sealing segment of the stent graft.

It should be understood that the above description is intended for illustrative purposes only, and is not intended to limit the scope of the present disclosure in any way. Thus, those skilled in the art will appreciate that other aspects of the disclosure can be obtained from a study of the drawings, the disclosure and the appended claims.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in United States patent application number 15/709,638, from which this application claims priority, and in the abstract accompanying this application are incorporated herein by reference.

### List of Elements

- 10.: aorta
- 11.: illiac artery
- 12.: established clot
- 14.: aneurism
- 15.: sac
- 16.: blood vessel
- 17.: blood vessel
- 18.: vessel wall
- 19.: implantation site
- 20.: stent graft
- 21.: stent
- 22.: fabric tube
- 23.: outer surface
- 24.: entrance opening
- 25.: exit opening
- 26.: entrance diameter
- 27.: sealing segment
- 30.: streamer
- 31.: attachment end
- 32.: coagulate end
- 33.: length dimension
- 34.: width dimension
- 35.: thickness dimension
- 40.: suture
- 41.: stent graft
- 42.: stent graft

## Claims

1. A stent graft comprising:
a stent;
a fabric tube supported by and attached to the stent;
a plurality of streamers that each includes an attachment end attached to an outer surface of the fabric tube and an unattached coagulant end that is free to move with respect to the fabric tube so as to promote coagulation of blood contacting the streamers after stent graft implantation; and
wherein each of the streamers has a length dimension greater than both a width dimension and a thickness dimension thereof.

2. A stent graft according to claim 1, wherein each of the streamers is formed of an extracellular matrix material.

3. A stent graft according to claim 2, wherein the extracellular matrix includes small intestine submucosa.

4. A stent graft according to any preceding claim, wherein each of the streamers comprises a hydrogel strip.

5. A stent graft according to any preceding claim, wherein each of the streamers includes embolic fibers.

6. A stent graft according to any preceding claim, wherein each of the streamers is a woven ribbon of the embolic fibers.

7. A stent graft according to any preceding claim, wherein the streamers includes a dissolvable coating operable to inhibit coagulation promotion prior to implantation.

8. A stent graft according to any preceding claim, wherein an end segment of the stent graft is a sealing segment for contacting a vessel wall at an implantation site; and
the sealing segment is free of streamer attachment, and all of the streamers are attached to the fabric tube spaced from the sealing segment.

9. A stent graft according to any preceding claim, including one entrance opening and two exit openings.

10. A stent graft according to any preceding claim, wherein the length dimension of the streamers is of a same order of magnitude as an entrance diameter of the fabric tube.

11. A stent graft according to any preceding claim, wherein the streamers comprise at least one of a bioscaffold, a hydrogel and embolic fibers.

12. A stent graft according to any preceding claim, wherein the streamers include a dissolvable coating to inhibit coagulation promotion prior to implantation through contact of blood with the at least one of bioscaffold, a hydrogel and embolic fibers.
